# EUROPEAN PATENT APPLICATION

(11) **EP 2 586 442 A1**
(43) Date of publication of application: **01.05.2013**
(21) Application number: 11798271.0
(22) Date of filing: 24.06.2011
(51) Int. Cl.: A61K 31/426, A61P 9/12, A61P 13/12, A61P 19/06

(54) **SUSTAINED-RELEASE THERAPEUTIC AGENT FOR HYPERTENSION AND RENAL DYSFUNCTION**

(30) Priority: 25.06.2010 JP 2010145056
(71) Applicant: Teijin Pharma Limited, Tokyo 100-0013 (JP)
(72) Inventor: SHIRAKURA, Takashi, Hino-shi Tokyo 191-0065 (JP); TAMURA, Mizuho, Hino-shi Tokyo 191-0065 (JP); TAKAHASHI, Yoshimasa, Hino-shi Tokyo 191-0065 (JP); KUWAHARA, Ippei, Sagamihara-shi Kanagawa 252-0131 (JP)
(74) Representative: Hallybone, Huw George
(86) International application number: PCT/JP2011/064569
(87) International publication number: WO 2011/162390

(57) **Abstract**

The present invention provides a therapeutic or preventive agent for hypertension or normal high blood pressure, and for renal dysfunction, that is more effective than existing drugs.

More specifically, the present invention provides a sustained-release pharmaceutical composition for treating or preventing hypertension or normal high blood pressure, and for renal dysfunction, comprising as an active ingredient a 2-phenylthiazole compound represented by the following formula (I) 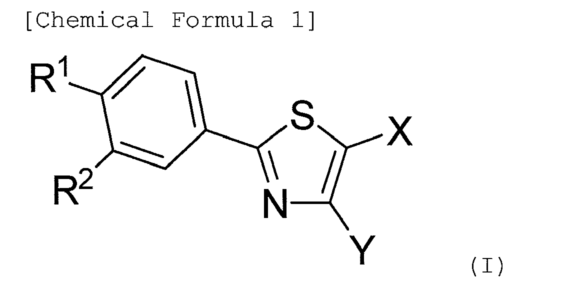 or a pharmaceutically acceptable salt thereof.

## Description

### [Technical Field]

The present invention relates to a sustained-release pharmaceutical composition for treating or preventing hypertension or normal high blood pressure, and to a therapeutic or preventive method using the same.

Furthermore, the present invention relates to a sustained-release pharmaceutical composition for treating or preventing renal dysfunction, and to a therapeutic or preventive method using the same.

### [Background Art]

Xanthine oxidase inhibitors have the effect of lowering blood uric acid levels by inhibiting uric acid synthesis, and thus ameliorate hyperuricemia and gout.

At the same time, it has been pointed out that hyperuricemia is associated with hypertension, and, indeed, it has been reported that hyperuricemia is a risk factor for the development of hypertension (Non-Patent Document 1). Therefore, xanthine oxidase inhibitors, which ameliorate hyperuricemia, could be potential therapeutics for hypertension.

In addition, it has also been pointed out that hyperuricemia is associated with renal dysfunction, and it has been reported, on the basis of animal experiments, that hyperuricemia plays a pathogenic role in the development and progression of renal dysfunction, and, on the basis of a clinical study, that hyperuricemia is a risk factor for poor prognosis of renal function (Non-Patent Documents 2, 3). Therefore, xanthine oxidase inhibitors, which ameliorate hyperuricemia, could be potential therapeutics for renal dysfunction.

On the other hand, xanthine oxidase inhibitors are known not only to inhibit uric acid synthesis but also to suppress the generation of reactive oxygen species (Non-Patent Document 4). It has been suggested that reactive oxygen species, which are cytotoxic, may be involved in the development of hypertension and renal dysfunction (Non-Patent Document 5). Therefore, xanthine oxidase inhibitors may have the effect of ameliorating hypertension and renal dysfunction without depending on the uric acid lowering action, as they inhibit the generation of reactive oxygen species.

The 2-Phenylthiazole compounds employed in the present invention, such as 2-(3-cyano-4-isobutyloxyphenyl)-4-methyl-5-thiazole carboxylic acid, are known to have the effect of lowering uric acid levels by inhibiting xanthine oxidase, and to be potential therapeutics for hyperuricemia and gout (Non-Patent Document 6). They are also known to have the effect of lowering blood pressure and to be potential therapeutics for hypertension, and to have the effect of preserving renal functions and to be potential therapeutics for renal dysfunction (Patent Documents 1, 2). However, it has not been known that these effects are remarkably enhanced by administering the above compounds in a sustained-release manner.

### [Prior Art Document]

[Patent Document 1] International Publication No. WO/2008/064015
[Patent Document 2] International Publication No. WO/2007/019153
[Non-Patent Document 1] Hypertension, 2006; 48: 1031-1036
[Non-Patent Document 2] American Journal of Kidney Diseases, 2004; 44: 642-650
[Non-Patent Document 3] Hypertension, 2003; 41: 1183-1190
[Non-Patent Document 4] Journal of Physiology, 2004; 555: 589-606
[Non-Patent Document 5] Journal of Nephrology, 2008; 21(2): 175-179
[Non-Patent Document 6] Arthritis and Rheumatism, 2005; 52: 916-923

### [Summary of Invention]

### [Problems to be Solved by the Invention]

It is an object of the present invention to provide a therapeutic or preventive agent for hypertension or normal high blood pressure that is more effective than existing drugs. It is also an object of the present invention to provide a method for treating or preventing hypertension or normal high blood pressure that is more effective than existing methods.

It is another object of the present invention to provide a therapeutic or preventive agent for renal dysfunction that is more effective than existing drugs. It is also another object of the present invention to provide a method for treating or preventing renal dysfunction that is more effective than existing methods.

### [Means for Solving the Problem]

As a result of conducting diligent studies for achieving the above objects, the present inventors have found that much greater medicinal effects are obtained by administering the 2-phenylthiazole compounds employed in the present invention in a sustained-release manner than by administering them in an immediate-release manner.

Thus, the present invention is directed to a sustained-release pharmaceutical composition for treating or preventing hypertension or normal high blood pressure, which comprises as an active ingredient a 2-phenylthiazole compound represented by the following formula ( I ) 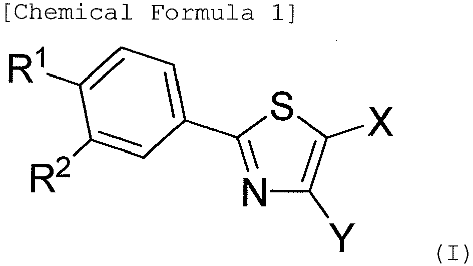 wherein
R¹ represents a C₁₋₈ alkoxy group, a morpholino group, a 4-methylpiperazin-1-yl group, or a piperidino group;
R² represents a nitro group or a cyano group;
X represents a carboxyl group or a C₂₋₇ alkoxycarbonyl group; and
Y represents a hydrogen atom or a C₁₋₆ alkyl group, or a pharmaceutically acceptable salt thereof.

The present invention is also directed to a sustained-release pharmaceutical composition for treating or preventing renal dysfunction, which comprises as an active ingredient a 2-phenylthiazole compound represented by formula (I) above or a pharmaceutically acceptable salt thereof.

In addition, the present invention is directed to a method for treating or preventing hypertension or normal high blood pressure, which comprises administrating a 2-phenylthiazole compound represented by formula (I) above or a pharmaceutically acceptable salt thereof, in an amount effective for the treatment or prevention of hypertension or normal high blood pressure, and in a sustained-release manner.

Furthermore, the present invention is directed to a method for treating or preventing renal dysfunction, which comprises administrating a 2-phenylthiazole compound represented by formula (I) above or a pharmaceutically acceptable salt thereof, in an amount effective for the treatment or prevention of renal dysfunction, and in a sustained-release manner.

### [Effect of the Invention]

According to the present invention, much more potent therapeutic or preventive effects can be obtained by administrating a 2-phenylthiazole compound or a pharmaceutically acceptable salt thereof in a sustained-release manner than by administrating it in an immediate-release manner.

### [Description of Embodiments]

The present invention employs a 2-phenylthiazole compound represented by the following formula (I): 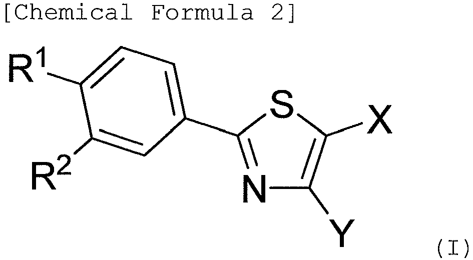 wherein
R¹ represents a C₁₋₈ alkoxy group, a morpholino group, a 4-methylpiperazin-1-yl group, or a piperidino group;
R² represents a nitro group or a cyano group;
X represents a carboxyl group or a C₂₋₇ alkoxycarbonyl group; and
Y represents a hydrogen atom or a C₁₋₆ alkyl group, or a pharmaceutically acceptable salt thereof. An example of the compound is 2-(3-cyano-4-isobutyloxyphenyl)-4-methyl-5-thiazole carboxylic acid, and such a compound can be produced by known methods, such as the one described in WO/92/09279.

If necessary, the compound represented by formula (I) above can be converted into pharmaceutically acceptable salts. Examples of such salts include: salts with inorganic acids such as hydrochloric acid, hydrobromic acid, hydroiodic acid, sulfuric acid, nitric acid, phosphoric acid, and carbonic acid; salts with organic acids such as formic acid, acetic acid, propionic acid, trifluoroacetic acid, phthalic acid, oxalic acid, malonic acid, succinic acid, fumaric acid, maleic acid, lactic acid, malic acid, tartaric acid, citric acid, benzoic acid, methanesulfonic acid, ethanesulfonic acid, benzenesulfonic acid, and p-toluenesulfonic acid; salts with amino acid such as lysine, arginine, ornithine, glutamic acid, and aspartic acid; salts with alkali metals such as sodium, potassium, and lithium; salts with alkaline-earth metals such as calcium and magnesium; salts with metals such as aluminum, zinc, and iron; salts with organic bases such as methylamine, ethylamine, t-octylamine, diethylamine, trimethylamine, triethylamine, ethylenediamine, piperidine, piperazine, pyridine, picoline, ethanolamine, diethanolamine, triethanolamine, cyclohexylamine, dicyclohexylamine, N-methylglucamine, tris(hydroxymethyl)aminomethane, and N,N'-dibenzylethylenediamine; ammonium salts, and the like.

The dosage of the active ingredient of the present invention is an amount that is effective for the treatment or prevention of hypertension or normal high blood pressure, hypertension or normal high blood pressure with hyperuricemia or gout, hypertension or normal high blood pressure with renal dysfunction, renal dysfunction, renal dysfunction with hyperuricemia or gout, or renal dysfunction with hypertension or normal high blood pressure. The dosage may vary depending on the age and body weight of the patient, type of concurrent treatment, frequency of treatment, nature of the effect desired, mode of administration, or the like.

The therapeutic or preventive agent of the present invention may be administrated daily or intermittently, and the daily dose can be given all at once or divided into 2 or 3 doses.

In the context of the present invention, the term "sustained-release pharmaceutical composition" refers to a composition that can maintain an effective blood level for a long period of time, for example, for 13 hours or more, preferably for 16 hours or more, and more preferably for 18 hours or more.

In the context of the present invention, the term "administering...in a sustained-release manner" means that administration is provided in such a manner that an effective blood level is maintained for a long period of time, for example, for 13 hours or more, preferably for 16 hours, and more preferably for 18 hours or more. Dosage forms of a sustained-release pharmaceutical composition and dosage forms for providing administration in a sustained-release manner include extended-release dosage forms.

The term "effective blood level" just mentioned above refers to an effective blood level with respect to the disease to be treated or prevented. The effective blood level can be determined experimentally or clinically by a person skilled in the art.

As such extended release dosage forms, any dosage form, such as solid preparation, semi-solid preparation, and liquid preparation, and even formulation for any administration route, such as oral formulation and parenteral formulation (injections, transdermal formulations, eye drops, suppositories, intranasal formulations, inhalants, and the like), can be used. Such preparations can be produced by known methods.

Generally, such extended-release dosage forms are prepared by adding a slow-release agent to additives commonly used to formulate pharmaceutical preparations. In the context of the present invention, the slow-release agent refers to an additive to be added to control the dissolution of the active ingredient from preparations in vivo. Examples of the slow-release agent may include water-soluble polymers such as hydroxypropylmethylcellulose, water-insoluble polymers such as ethylcellulose, enteric polymers such as (meth)acrylic acid copolymer, biodegradable polymers such as polylactate, and the like. The additives commonly used to formulate pharmaceutical preparations may include: excipients such as lactose, sucrose, glucose, corn starch, potato starch, crystalline cellulose, light anhydrous silicic acid, synthetic aluminum silicate, magnesium aluminometasilicate, and calcium hydrogen phosphate; binders such as crystalline cellulose, carboxymethyl cellulose, hydroxypropylcellulose, sodium carboxymethylcellulose, and polyvinylpyrrolidone; disintegrants such as starch, sodium carboxymethylcellulose, calcium carboxymethylcellulose, croscarmellose sodium, and sodium carboxymethyl starch; lubricants such as talc and stearates; coating agents such as hydroxymethylpropylcellulose, hydroxypropylmethylcellulose phthalate, and ethylcellulose; colorants; in the case of semi-solid preparations, bases such as white petrolatum; in the case of liquid preparations, solvents such as ethanol, solubilizing agents such as ethanol, preservatives such as p-hydroxybenzoate esters, isotonization agents such as glucose, buffering agents such as citric acid, antioxidants such as L-ascorbic acid, chelating agents such as EDTA, and suspending or emulsifying agents such as polysorbate 80, and the like. The extended-release dosage form, slow-release agent, and additives can be appropriately selected by a person skilled in the art, including, by way of example, matrix-type formulations containing an active ingredient of the present invention and a slow-release agent such as hydroxypropylmethylcellulose, in which the slow-release agent is distributed in a matrix throughout the formulation; granules in which a core particle containing crystalline cellulose and the like is coated with a layer containing an active ingredient of the present invention and a slow-release agent such as hydroxypropylmethylcellulose; film-controlled type tablets in which a plain tablet containing an active ingredient of the present invention and an excipient such as lactose is coated with a slow-release agent such as ethylcellulose.

Hypertension, in the context of the present invention, is defined as a systolic blood pressure of 140 mmHg or above and/or a diastolic blood pressure of 90 mmHg or above. Normal high blood pressure, in the context of the present invention, is defined as a systolic blood pressure of 130 mmHg or above but less than 140 mmHg and/or a diastolic blood pressure of 85 mmHg or above but less than 90 mmHg.

In addition, hypertension, in the context of the present invention, includes hypertension or normal high blood pressure with hyperuricemia or gout, and hypertension or normal high blood pressure with renal dysfunction.

Renal dysfunction, in the context of the present invention, is defined as, regardless of the type of renal diseases causing it, conditions in which proteins or albumin are persistently excreted into urine at above normal levels; in mild cases, for example, conditions in which urinary albumin excretion levels are 30 mg/day or more, 20 µg/min or more, or 30 mg/g creatinine (mg/gCr, urinary albumin/creatinine ratio) or more, and/or conditions in which estimated glomerular filtration rates (eGFR), calculated from serum creatinine values according to the standards of each country, are less than or equal to 60 mL/min/1.73 m²; in moderate to severe cases, for example, conditions in which urinary protein excretion levels are 0.5 g/day or more, or urinary albumin excretion levels are 300 mg/day or more, 200 µg/min or more, or 300 mg/g creatinine (mg/gCr, urinary albumin/creatinine ratio) or more, and/or conditions in which estimated glomerular filtration rates (eGFR), calculated from serum creatinine values according to the standards of each country, are less than or equal to 30 mL/min/1.73 m².

By treating or preventing renal dysfunction using the present invention, diseases that cause renal dysfunction, such as diabetic nephropathy, chronic glomerulonephritis, nephrotic syndrome, IgA nephropathy, etc. can be treated or prevented.

In addition, renal dysfunction, in the context of the present invention, includes renal dysfunction with hyperuricemia or gout, and renal dysfunction with hypertension or normal high blood pressure.

### EXAMPLES

### Example 1

The effects of the immediate-release and sustained-release administrations of febuxostat in a spontaneous hypertension model were ascertained. Male spontaneously hypertensive rats (SHR) aged 12 weeks were subjected to quarantine for a week or more before used as subjects. From 13 weeks of age, habituation to blood pressure measurement with a Tail-Cuff sphygmomanometer (BP-2000, Visitech systems, Napa Place, NC, USA) was performed for two weeks. At 15 weeks of age after the completion of the habituation, blood pressure was measured, and the subjects were divided into three groups (10 subjects in each group), such that the systolic blood pressures were evenly distributed. Subsequently, the administrations were started. Group 1, a control group, was given tap water. Group 2 was given febuxostat in drinking water. Group 3 was given febuxostat by oral gavage.

The administration in drinking water was performed by giving the subjects access to an aqueous solution of febuxostat ad libitum. In this manner, prolonged drug exposure to the blood, i.e, sustained-release administration can be accomplished, as compared to a single oral administration. On the other hand, the oral administration was performed by giving a suspension of febuxostat in 0.5% MC solution (with a concentration of febuxostat of 2 mg/mL) at 5 mL/kg by oral gavage. The blood level of the drug rapidly increases after the administration and diclines soon. Thus, immediate-release administration can be accomplished in this manner. After 18 days of administration, systolic blood pressure was measured by the Tail-Cuff method. Table 1 shows the constitution of groups, dosages of febuxostat, and measurements of systolic blood pressure (mean ± standard error: mmHg).

This evaluation confirmed that blood pressure was more strongly lowered by administering febuxostat in a sustained-release manner than by administering it in an immediate-release manner.

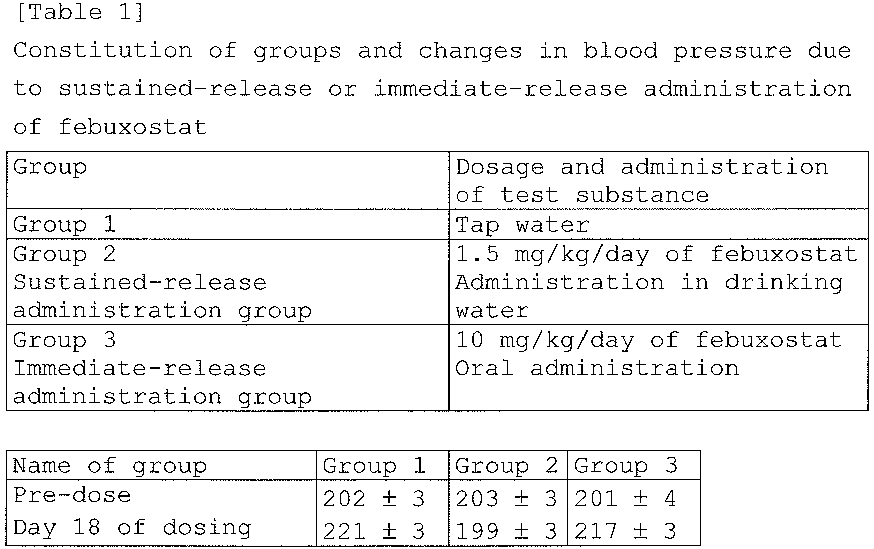

### Example 2

The effects of the immediate-release and sustained-release administrations of febuxostat in an adriamycin-induced nephropathy mouse model were ascertained. The adriamycin-induced nephropathy model is known as a renal dysfunction model in which lesions occur in glomerular epithelial cells and proteinuria is observed.

Male BALB/cAnNCrlCrlj mice aged 7 weeks were subjected to quarantine for a week or more before used as subjects.

At 8 weeks of age, a 24-hour urine collection was performed and the concentration of albumin in the collected urine was measured by ELISA. The subjects were divided into three groups, such that the amounts of albumin excretion were evenly distributed. Subsequently, the administrations were started. Group 1, a control group, was given tap water. Group 2 was given febuxostat in drinking water. Group 3 was given febuxostat by oral gavage.

The administration in drinking water was performed by giving the subjects access to an aqueous solution of febuxostat ad libitum. In this manner, prolonged drug exposure, i.e, sustained-release administration can be accomplished, as compared to a single oral administration. On the other hand, the oral administration was performed by giving a suspension of febuxostat in 0.5% MC solution by oral gavage via a tube. The blood level of the drug rapidly increases after the administration and diclines soon. Thus, immediate-release administration can be accomplished in this manner. Following the initiation of dosing on the first day, adriamycin was given to mice via tail vein at 10 mg/kg. Then, the administrations were conducted for 5 consecutive days. A 24-hour urine collection was performed 5 days after onset of drug administration and the amount of albumin in the collected urine was measured as previously described. Table 2 shows the constitution of groups, dosages of febuxostat, and amounts of albumin in the urine of the respective groups, corrected for urine volume (mean ± standard error: mg/day).

This evaluation confirmed that renal dysfunction was more effectively ameliorated by administering febuxostat in a sustained-manner than by administering it in an immediate-release manner.

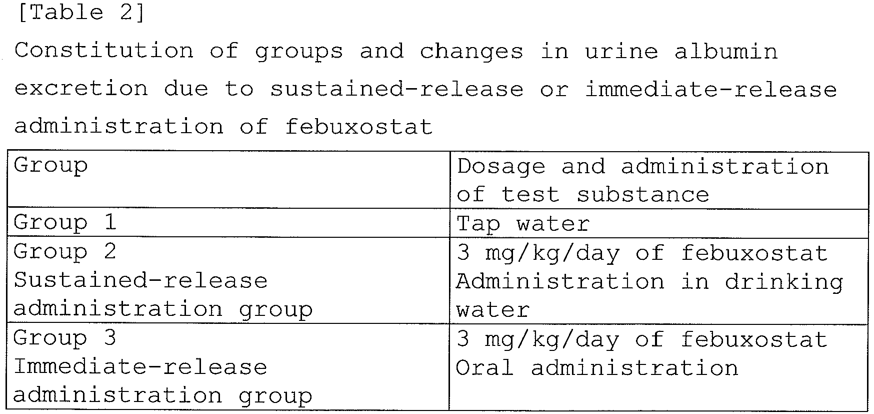

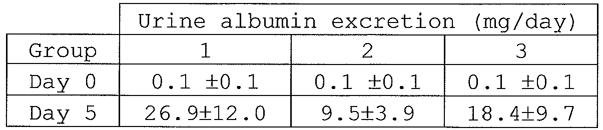

### [Industrial Applicability]

The present invention can be used for the treatment or prevention of hypertension or normal high blood pressure. In addition, the present invention can be used for the treatment or prevention of renal dysfunction.

## Claims

1. A sustained-release pharmaceutical composition for treating or preventing hypertension or normal high blood pressure, comprising as an active ingredient a 2-phenylthiazole compound represented by the following formula (I): 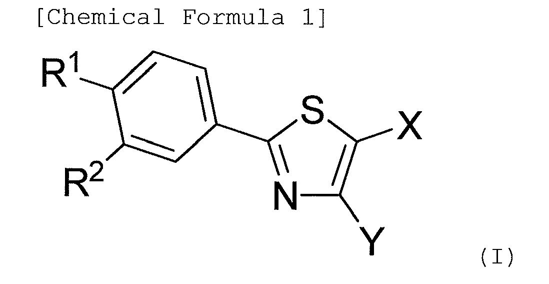 wherein
R¹ represents a C₁₋₈ alkoxy group, a morpholino group, a 4-methylpiperazin-1-yl group, or a piperidino group;
R² represents a nitro group or a cyano group;
X represents a carboxyl group or a C₂₋₇ alkoxycarbonyl group; and
Y represents a hydrogen atom or a C₁₋₆ alkyl group, or a pharmaceutically acceptable salt thereof.

2. The sustained-release pharmaceutical composition according to Claim 1, wherein the hypertension or normal high blood pressure is hypertension or normal high blood pressure with hyperuricemia or gout.

3. The sustained-release pharmaceutical composition according to Claim 1, wherein the hypertension or normal high blood pressure is hypertension or normal high blood pressure with renal dysfunction.

4. A sustained-release pharmaceutical composition for treating or preventing renal dysfunction, comprising as an active ingredient a 2-phenylthiazole compound represented by the formula (I) or a pharmaceutically acceptable salt thereof.

5. The sustained-release pharmaceutical composition according to Claim 4, wherein the renal dysfunction is renal dysfunction with hyperuricemia or gout.

6. The sustained-release pharmaceutical composition according to Claim 4, wherein the renal dysfunction is renal dysfunction with hypertension or normal high blood pressure.

7. The sustained-release pharmaceutical composition according to any one of Claims 4 to 6, wherein the renal dysfunction is diabetic nephropathy, chronic glomerulonephritis, nephrotic syndrome, or IgA nephropathy.

8. The sustained-release pharmaceutical composition according to any one of Claims 1 to 7, wherein the 2-phenylthiazole compound represented by the formula (I) is 2-(3-cyano-4-isobutyloxyphenyl)-4-methyl-5-thiazole carboxylic acid or a pharmaceutically acceptable salt thereof.

9. A method for treating or preventing hypertension or normal high blood pressure, the method comprising administrating, in an amount effective for the treatment or prevention of hypertension or normal high blood pressure, and in a sustained-release manner, a 2-phenylthiazole compound represented by the following formula (I): 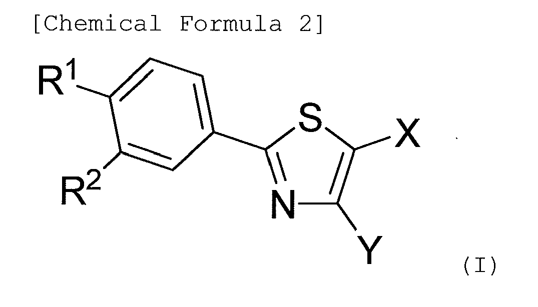 wherein
R¹ represents a C₁₋₈ alkoxy group, a morpholino group, a 4-methylpiperazin-1-yl group, or a piperidino group;
R² represents a nitro group or a cyano group;
X represents a carboxyl group or a C₂₋₇ alkoxycarbonyl group; and
Y represents a hydrogen atom or a C₁₋₆ alkyl group, or a pharmaceutically acceptable salt thereof.

10. The therapeutic or preventive method according to Claim 9, wherein the hypertension or normal high blood pressure is hypertension or normal high blood pressure with hyperuricemia or gout.

11. The therapeutic or preventive method according to Claim 9, wherein the hypertension or normal high blood pressure is hypertension or normal high blood pressure with renal dysfunction.

12. A method for treating or preventing renal dysfunction, the method comprising administrating, in an amount effective for the treatment or prevention of renal dysfunction, and in a sustained-release manner, a 2-phenylthiazole compound represented by the formula (I) or a pharmaceutically acceptable salt thereof.

13. The therapeutic or preventive method according to Claim 12, wherein the renal dysfunction is renal dysfunction with hyperuricemia or gout.

14. The therapeutic or preventive method according to Claim 12, wherein the renal dysfunction is renal dysfunction with hypertension or normal high blood pressure.

15. The therapeutic or preventive method according to any one of Claims 12 to 14, wherein the renal dysfunction is diabetic nephropathy, chronic glomerulonephritis, nephrotic syndrome, or IgA nephropathy.

16. The therapeutic or preventive method according to any one of Claims 9 to 15, wherein the 2-phenylthiazole compound represented by the formula (I) is 2-(3-cyano-4-isobutyloxyphenyl)-4-methyl-5-thiazole carboxylic acid or a pharmaceutically acceptable salt thereof.
